# EUROPEAN PATENT APPLICATION

(11) **EP 4 596 593 A1**
(43) Date of publication of application: **06.08.2025**
(21) Application number: 23869510.0
(22) Date of filing: 24.03.2023
(51) Int. Cl.: C08F 226/06, C08F 212/36, C08F 212/08, C07H 1/00

(54) **IMMOBILIZED 4,5-DICYANOIMIDAZOLE, PREPARATION METHOD THEREFOR, AND USE THEREOF**

(30) Priority: 29.09.2022 CN 202211203439
(71) Applicant: Liaoning Asymchem Laboratories Co., Ltd., Fuxin, Liaoning 123129 (CN)
(72) Inventor: HONG, Hao, Morrisville North Carolina 27560 (US); JAMES, Gage, Morrisville North Carolina 27560 (US); XIAO, Yi, Fuxin, Liaoning 123129 (CN); PAN, Long, Fuxin, Liaoning 123129 (CN); SUN, Yuchen, Fuxin, Liaoning 123129 (CN); ZHANG, Hangfei, Fuxin, Liaoning 123129 (CN)
(74) Representative: Zacco GmbH
(86) International application number: PCT/CN2023/083865
(87) International publication number: WO 2024/066250

(57) **Abstract**

The present disclosure provides an immobilized 4,5-dicyanoimidazole, and a preparation method and application thereof. The immobilized 4,5-dicyanoimidazole is obtained through a free radical copolymerization reaction of a first comonomer, a second comonomer, and a third comonomer. The first comonomer is divinyl benzene or ethylene glycol dimethacrylate. The second comonomer is styrene or methyl methacrylate. The third comonomer is 2-vinyl-4,5-dicyanoimidazole. The problem that a 4,5-dicyanoimidazole is difficultly separated from a reaction system after an activation coupling reaction in the prior art can be solved. Therefore, the present disclosure is suitable for the field of immobilization of the 4,5-dicyanoimidazole.

## Description

### Technical Field

The present disclosure relates to the field of immobilization of the 4,5-dicyanoimidazole, and specifically, to an immobilized 4,5-dicyanoimidazole, and a preparation method and application thereof.

### Background

Small nucleic acids are oligonucleotide molecules that include small interfering nucleic acids (siRNAs), Antisense Oligonucleotides (ASOs), microRNAs (miRNAs), and nucleic acid aptamers. Small nucleic acid drugs are composed of nucleotides, and belong to a completely new category of drugs that are completely different from small molecule drugs and antibody drugs. Compared to traditional chemical drug molecules, the small nucleic acid drugs have the advantages of being strong in targeted specificity, efficient, long-acting in drug action, simple in drug design, rich in candidate target, etc. Main small nucleic acid drugs are siRNA drugs and ASO drugs, which mainly act on mRNAs of cytoplasm. Through base complementary recognition and repression of target mRNAs, regulation of protein expression is realized, thereby achieving the purpose of treating diseases.

In recent years, the demand for the small nucleic acid drugs is gradually increasing. In 1998, the U.S. Food and Drug Administration (FDA) approved the marketing of the first small nucleic acid (oligonucleotide) drug Vitravene for the treatment of cytomegalovirus retinitis. With the increasing success of clinical projects and the potential market demand for oligonucleotide-based drugs, it is important to develop safe, environmentally-clean and cost-effective synthesis methods for these molecules. During the synthesis of the oligonucleotide, a key step is the coupling of ribose and deoxyribose portions with phosphoramidite compounds. In a laboratory, a phosphorylation reagent frequently used by scientific research personnel is 2-cyanoethyl-N,N-diisopropylchlorophosphoramidite (as shown in Formula A), which is relatively reactive and does not require additional activating reagents to react with hydroxyl on a glycosyl furan ring for coupling. In the laboratory, the reaction may be performed by directly using an automated solid-phase synthesis instrument.

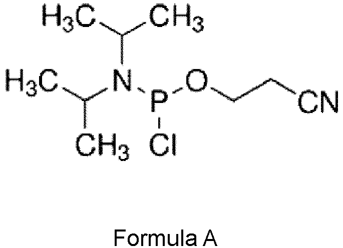

However, the 2-cyanoethyl-N,N-diisopropylchlorophosphoramidite is expensive in price, unstable at room temperature, and easy to explode when being heated. The use of such raw material increases the cost of industrial production and brings potential risks. A cheap and safer 2-cyanoethyl-N,N,N',N'-tertraisopropylphosphorodiamidite (as shown in Formula B) may be used as an alternative of the above raw material. However, the raw material is low in reaction activity, and an amphoteric activation reagent such as tetrazole and 4,5-dicyanoimidazole (4,5-DCI) is required for activation. The tetrazole is costly and toxic, and is also a potentially explosive substance that is difficult to scale up for industrial applications. As a safe active agent, the 4,5-DCI is widely used in many phosphorylation reactions, but its main disadvantage is that it is insoluble in water and is not easily removed after the reaction (on the contrary, the tetrazole is water-soluble and may be removed through water washing), and it needs to be passed through a silica gel column. Therefore, there is an adverse effect on system scaling up. The 4,5-DCI is still difficult to be completely replaced at the current stage due to its safety, cheap availability, and good reaction activity.

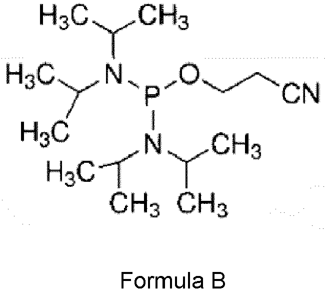

### Summary

The present disclosure is mainly intended to provide an immobilized 4,5-dicyanoimidazole, and a preparation method and application thereof, so as to solve the problem that a 4,5-dicyanoimidazole is difficultly separated from a reaction system after an activation coupling reaction in the prior art.

In order to implement the above objective, a first aspect of the present disclosure provides an immobilized 4,5-dicyanoimidazole. The immobilized 4,5-dicyanoimidazole is obtained through a free radical copolymerization reaction of a first comonomer, a second comonomer, and a third comonomer. The first comonomer is divinylbenzene or ethylene glycol dimethacrylate. The second comonomer is styrene or methyl methacrylate. The third comonomer is 2-vinyl-4,5-dicyanoimidazole.

Further, in the immobilized 4,5-dicyanoimidazole, a mass ratio of the first comonomer, the second comonomer and the third comonomer is (10-50): (10-50): (10-80); a ratio of the total mass of the first comonomer and the second comonomer to the mass of the third comonomer is ≤9:1; preferably, the mass ratio of the first comonomer, the second comonomer, and the third comonomer is (20-30): (20-30): (40-60); and more preferably, the mass ratio of the first comonomer, the second comonomer, and the third comonomer is 1:1:2.

Further, the solubility of the immobilized 4,5-dicyanoimidazole in all of N,N-dimethylformamide, ether, acetonitrile, alcohol, ester and chloralkane is < 1 g/L; and preferably, an infrared spectrum of the immobilized 4,5-dicyanoimidazole has the following characteristic absorption: 3149-2488 cm⁻¹ of multiple peaks, 2242, 1911, 1728, 1644, 1574, 1510, and 1370 cm⁻¹; and a method for preparing an infrared test sample of the immobilized 4,5-dicyanoimidazole is a potassium bromide pellet method.

In order to implement the above objective, a second aspect of the present disclosure provides a method for preparing an immobilized 4,5-dicyanoimidazole. The method includes: applying a first comonomer and a second comonomer to undergo a free radical copolymerization reaction on 2-ethenyl-4,5-dicyanoimidazole, wherein the first comonomer is divinylbenzene or ethylene glycol dimethacrylate, and the second comonomer is styrene or methyl methacrylate.

Further, in the free radical copolymerization reaction, a feeding mass of the first comonomer is 10-50 parts, a feeding mass of the second comonomer is 10-50 parts, and a sum of the feeding masses of the first comonomer and the second comonomer does not exceed 90 parts; a feeding mass of the 2-ethenyl-4,5-dicyanoimidazole is 10-80 parts; preferably, the feeding mass of the first comonomer is 20-30 parts, the feeding mass of the second comonomer is 20-30 parts, and the feeding mass of the 2-ethenyl-4,5-dicyanoimidazole is 40-60 parts; and more preferably, the feeding mass of the first comonomer is 25 parts, the feeding mass of the second comonomer is 25 parts, and the feeding mass of the 2-ethenyl-4,5-dicyanoimidazole is 50 parts.

Further, the first comonomer, the second comonomer, and the 2-ethenyl-4,5-dicyanoimidazole are put in a reaction solvent to undergo the free radical copolymerization reaction, wherein the reaction solvent includes one or more of tert-butyl methyl ether, acetonitrile or N,N-dimethylformamide; and preferably, the free radical copolymerization reaction is undergone in an atmosphere of nitrogen or a rare gas.

Further, a radical initiator of the free radical copolymerization reaction includes one or more of 2,2'-azobis(2-methylpropionitrile), 2,2'-azobis(2,4-dimethyl)valeronitrile, or benzoyl peroxide; preferably, a reaction temperature of the free radical copolymerization reaction ranges from 65 °C to 90 °C; and preferably, a reaction time for the free radical copolymerization reaction ranges from 10 h to 20 h.

In order to implement the above objective, a third aspect of the present disclosure provides a coupling method. The coupling method includes: using the above immobilized 4,5-dicyanoimidazole or the immobilized 4,5-dicyanoimidazole prepared by the above method for preparing an immobilized 4,5-dicyanoimidazole to activate phosphoramide and hydroxyl for coupling.

Further, the immobilized 4,5-dicyanoimidazole is used to activate the phosphoramide and the hydroxyl to undergo a coupling reaction, and a conversion rate of the coupling reaction ranges from 90% to 94%; preferably, a compound carrying the hydroxyl includes a nucleoside protected by dimethoxytrityl or a deoxynucleoside protected by the dimethoxytrityl; preferably, a compound carrying the phosphoramide includes a phosphosamide compound; and preferably, the phosphosamide compound includes 2-cyanoethyl-N,N,N',N'-tertraisopropylphosphorodiamidite.

Further, the coupling method includes using the immobilized 4,5-dicyanoimidazole to undergo a continuous coupling reaction.

In order to implement the above objective, a fourth aspect of the present disclosure provides an application of the immobilized 4,5-dicyanoimidazole, or the method for preparing an immobilized 4,5-dicyanoimidazole, or the coupling method in a coupling reaction of a phosphoramide and a hydroxyl, and/or, an oligonucleotide synthesis.

Through the application of the technical solutions of the present disclosure, the 4,5-dicyanoimidazole is prepared into the immobilized 4,5-dicyanoimidazole, and the immobilized 4,5-dicyanoimidazole is highly crosslinked and insoluble in common organic solvents, and could be removed from a reaction system through simple filtration means after an activation coupling reaction.

### Detailed Description of the Embodiments

It is to be noted that the embodiments in the present application and the features in the embodiments may be combined with one another without conflict. The present disclosure will be described below in detail with reference to the embodiments.

As mentioned in the Background, as a safe active agent, the 4,5-DCI is widely used in many phosphorylation reactions, but its main disadvantage is that it is soluble in organic solvents and insoluble in water, and is not easy to remove after a reaction, thus, the 4,5-DCI is difficult to use in the scaled-up reaction system. Therefore, in the present application, the inventor attempts to prepare an immobilized 4,5-dicyanoimidazole. With a free radical copolymerization reaction, 2-ethenyl-4,5-dicyanoimidazole is prepared into the immobilized 4,5-dicyanoimidazole, facilitating the application in the phosphorylation reaction. Therefore, a series of protective solutions of the present application are proposed.

A first typical implementation of the present application provides an immobilized 4,5-dicyanoimidazole. The immobilized 4,5-dicyanoimidazole is obtained through a free radical copolymerization reaction of a first comonomer, a second comonomer, and a third comonomer. The first comonomer is divinylbenzene or ethylene glycol dimethacrylate. The second comonomer is styrene or methyl methacrylate. The third comonomer is 2-vinyl-4,5-dicyanoimidazole.

In a preferred embodiment, in the immobilized 4,5-dicyanoimidazole, a mass ratio of the first comonomer, the second comonomer and the third comonomer is (10-50): (10-50): (10-80); a ratio of the total mass of the first comonomer and the second comonomer to the mass of the third comonomer is less than or equal to 9:1; preferably, the mass ratio of the first comonomer, the second comonomer, and the third comonomer is (20-30): (20-30): (40-60); and more preferably, the mass ratio of the first comonomer, the second comonomer, and the third comonomer is 1:1:2.

In a preferred embodiment, the solubility of the immobilized 4,5-dicyanoimidazole in all of N,N-dimethylformamide, ether, acetonitrile, alcohol, ester and chloralkane is less than 1 g/L; and preferably, an infrared spectrum of the immobilized 4,5-dicyanoimidazole has the following characteristic absorption: 3149-2488 (multiple peaks), 2242, 1911, 1728, 1644, 1574, 1510, and 1370 cm⁻¹. A method for preparing an infrared test sample of the immobilized 4,5-dicyanoimidazole is a potassium bromide pellet method. The potassium bromide pellet method is a common method for preparing an infrared sample in the prior art.

The immobilized 4,5-dicyanoimidazole is a random copolymer obtained by the free radical copolymerization reaction. The solubility of the immobilized 4,5-dicyanoimidazole in the above organic solvent, as well as monomers or mixtures of other commonly-used organic solvents is less than 1 g/L, and even less than 0.1 g/L or 0.01 g/L, such that after the immobilized 4,5-dicyanoimidazole is used to active the subsequent reaction, the immobilized 4,5-dicyanoimidazole can be removed through simple filtration, and recycled in the reaction. The mass of the third comonomer in the immobilized 4,5-dicyanoimidazole may be represented with a method for determining nitrogen elements in the prior art.

A second typical implementation of the present application provides a method for preparing an immobilized 4,5-dicyanoimidazole. The preparation method includes: applying a first comonomer and a second comonomer to undergo a free radical copolymerization reaction on 2-ethenyl-4,5-dicyanoimidazole, where the first comonomer is divinylbenzene or ethylene glycol dimethacrylate, and the second comonomer is styrene or methyl methacrylate.

The inventor screens various crosslinking agents commonly used in the prior art, and finds that the immobilized 4,5-dicyanoimidazole with good performance can be obtained with divinylbenzene or ethylene glycol dimethacrylate, and styrene or methyl methacrylate, as the crosslinking agents to perform the free radical copolymerization reaction on the 2-ethenyl-4,5-dicyanoimidazole. The immobilized 4,5-dicyanoimidazole has poor solubility in the organic solvent, and cannot be soluble in any common organic solvents and mixtures of the organic solvents. Therefore, after the immobilized 4,5-dicyanoimidazole is used to active the subsequent reaction, the immobilized 4,5-dicyanoimidazole can be removed through simple filtration, and recycled in the reaction.

In a preferred embodiment, in the free radical copolymerization reaction, a feeding mass of the first comonomer is 10-50 parts, a feeding mass of the second comonomer is 10-50 parts, and a sum of the feeding masses of the first comonomer and the second comonomer does not exceed 90 parts; a feeding mass of the 2-ethenyl-4,5-dicyanoimidazole is 10-80 parts; preferably, the feeding mass of the first comonomer is 20-30 parts, the feeding mass of the second comonomer is 20-30 parts, and the feeding mass of the 2-ethenyl-4,5-dicyanoimidazole is 40-60 parts; and more preferably, the feeding mass of the first comonomer is 25 parts, the feeding mass of the second comonomer is 25 parts, and the feeding mass of the 2-ethenyl-4,5-dicyanoimidazole is 50 parts.

In the free radical copolymerization reaction, by adjusting the feeding amounts of the first comonomer and second comonomer, the preparation product immobilized 4,5-dicyanoimidazole with an excellent physical property can be obtained. The amount of the first comonomer used can affect a degree of crosslinking of a polymer product. Under the feeding amount, the degree of crosslinking is improved, and more importantly, the solubility of the polymer product obtained through preparation in the organic solvent can be reduced, facilitating subsequent removing by means of simple filtration, thereby realizing recycling. If the amount of the first comonomer added is too low, the degree of crosslinking of the product may be reduced accordingly, and the solubility of the polymer product obtained through preparation in the organic solvent increases, which is difficult to achieve the effect of simple removing and recycling. If the amount of the first comonomer added is too much, the percentage of the active ingredient 4,5-dicyanoimidazole in the final product may be reduced, affecting catalytic activity. Furthermore, excessive first comonomer added also easily causes the polymer product to be difficult to swell in the organic solvent during subsequent use, affecting catalytic activity. In the free radical copolymerization reaction, the amount of the second comonomer added facilitates adjustment of the formability of the polymer product. If the amount of the second comonomer added is too low, a polymer is not easy to mold. The feeding amount of the 2-ethenyl-4,5-dicyanoimidazole also affects the performance of the polymer. If the feeding amount of the 2-ethenyl-4,5-dicyanoimidazole is too low, the catalytic activity of the product reduces, and if the feeding amount is too much, the degree of crosslinking of the product reduces, the solubility in the organic solvent increases, and it is difficult to meet the requirements of recycling.

In a preferred embodiment, the first comonomer, the second comonomer, and the 2-ethenyl-4,5-dicyanoimidazole are put in a reaction solvent to undergo the free radical copolymerization reaction, where the reaction solvent includes, but is not limited to, one or more of tert-butyl methyl ether, acetonitrile or N,N-dimethylformamide; and preferably, the free radical copolymerization reaction is undergone in an atmosphere of nitrogen or a rare gas.

The free radical copolymerization reaction may be performed in the organic solvent or other commonly-used organic solvents. The first comonomer, the second comonomer, and the 2-ethenyl-4,5-dicyanoimidazole all can be soluble in the organic solvents, while the immobilized 4,5-dicyanoimidazole generated after the free radical copolymerization reaction has low solubility in the above reaction solvents, and insoluble polymers are generated during the reaction, such that while promoting the reaction in a positive direction, it also facilitates the isolation and purification of the product.

In a preferred embodiment, a radical initiator of the free radical copolymerization reaction includes one or more of 2,2'-azobis(2-methylpropionitrile), 2,2'-azobis(2,4-dimethyl)valeronitrile, or benzoyl peroxide; preferably, a reaction temperature of the free radical copolymerization reaction ranges from 65 °C to 90 °C; and preferably, a reaction time for the free radical copolymerization reaction ranges from 10 h to 20 h.

With the radical initiator, free radicals can be generated through decomposition after being heated, thereby triggering the free radical copolymerization reaction. In the free radical copolymerization reaction, a reaction temperature is 65-90 °C, and a reaction time is 10-20 h. A decomposition start temperature of the radical initiator such as 2,2'-azobis(2-methylpropionitrile) is about 64 °C, and a decomposition speed may be too fast when exceeding 85 °C and lead to violent polymerization. Such that the free radical copolymerization reaction is performed within the above temperature range, and the free radical copolymerization reaction is high in conversion rate, fast in reaction, and not easy to generate side products.

Since components such as a water phase, a dispersant, and the like are not added, the copolymerization reaction belongs to precipitated copolymerization, a product obtained through copolymerization is in the form of micro-fine powder, with a particle size of tens of microns. The reaction is performed at a fixed time. Once the reaction time is complete, the generated product is filtered, and there is no need to determine if the reaction has ended.

A third typical implementation of the present application provides a coupling method. The coupling method includes: using the immobilized 4,5-dicyanoimidazole prepared by the method for preparing an immobilized 4,5-dicyanoimidazole, or the above immobilized 4,5-dicyanoimidazole to activate phosphoramide and hydroxyl for coupling.

In a preferred embodiment, the immobilized 4,5-dicyanoimidazole is used to activate the phosphoramide and the hydroxyl to undergo a coupling reaction, and a conversion rate of the coupling reaction ranges from 90% to 94%; preferably, a compound carrying the hydroxyl includes nucleoside protected by dimethoxytrityl (DMTr group) or deoxynucleoside protected by the DMTr group; preferably, a compound carrying the phosphoramide includes a phosphosamide compound; and preferably, the phosphosamide compound includes 2-cyanoethyl-N, N, N', N'-tertraisopropylphosphorodiamidite.

With the immobilized 4,5-dicyanoimidazole, the coupling reaction of the phosphoramide and the hydroxyl can be activated, so as to complete a key synthesis step of products such as oligonucleotides having large commercial values. With the immobilized 4,5-dicyanoimidazole, cheap reaction raw materials can be catalyzed to undergo the coupling reaction, and a coupling reaction rate greater than 90% is reached. The insoluble nature of the immobilized 4,5-dicyanoimidazole in common organic solvents also reduces the post-treatment cost of the coupling reaction. The immobilized 4,5-dicyanoimidazole can be separated from the reaction system with physical methods such as filtration, such that while reducing the separation cost, the immobilized 4,5-dicyanoimidazole can also be recycled, thereby further reducing production costs.

In a preferred embodiment, the coupling method includes using the immobilized 4,5-dicyanoimidazole to undergo a continuous coupling reaction.

With the insoluble nature of the immobilized 4,5-dicyanoimidazole in the organic solvents, the immobilized 4,5-dicyanoimidazole may be fixed in a continuous reaction apparatus. Reaction substrates are soluble in the reaction solvents, are in contact with the immobilized 4,5-dicyanoimidazole when flowing through the continuous reaction apparatus and complete the reaction, such that continuous industrial mass production can be realized without performing separation such as filtration, thereby further reducing the purification cost of the products.

A fourth typical implementation of the present application provides an application of the immobilized 4,5-dicyanoimidazole, or the method for preparing an immobilized 4,5-dicyanoimidazole, or the coupling method in a coupling reaction of phosphoramide and hydroxyl and/or oligonucleotide synthesis.

The beneficial effects of the present application are further described in detail below with reference to specific embodiments.

### Embodiment 1

Preparation of immobilized 4,5-dicyanoimidazole.

Under a nitrogen atmosphere, 0.5 g of divinylbenzene, 0.5 g of styrene, and 1 g of 2-ethenyl-4,5-dicyanoimidazole (2-ethenyl-4,5-DCI) were put into a 25 mL two-necked flask, 10 mL of N,N-dimethylformamide (DMF) was added as a solvent, and 25 mg of 2,2'-azobis(2-methylpropionitrile) (AIBN) was added ultimately. A temperature was heated to 70 °C, and a system reaction was performed for 16 h. After overnight, heating was stopped. A product was filtered, washed with ethanol, and dried, so as to obtain 1.47 g of the product, and the obtained product was named as C-1. The product had the following characteristic absorption in an infrared spectrum: 3149-2488 cm⁻¹ of multiple peaks, 2242, 1911, 1728, 1644, 1574, 1510, and 1370 cm⁻¹. The product was crosslinking, insoluble in common solvents, and included, but was not limited to, methanol, ethanol, acetonitrile, toluene, dichloromethane, ether, DMF, DMSO, ethyl acetate, etc. 0.1 g of the product was placed for 10 min in the above solvents, respectively. Washing, drying, and weighing were performed before and after placing. No change was observed with naked eyes. A mass difference before and after as a percentage of mass (i.e., a mass loss) was less than 1%.

A coupling reaction activated by the immobilized 4,5-dicyanoimidazole included the following.
1. A coupling reaction of a 5-methylcytidine derivative and 2-cyanoethyl-N,N,N',N'-tertraisopropylphosphorodiamidite activated by the immobilized 4,5-dicyanoimidazole (immobilized 4,5-DCI, C-1). 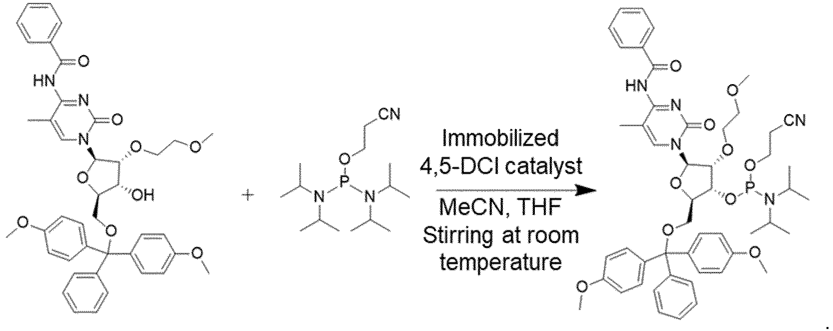 100 mg of immobilized 4,5-DCI resin (immobilized 4,5-dicyanoimidazole) was taken and added to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methylcytidine derivative was 94%.
2. A coupling reaction of a 5-methyluridine derivative and 2-cyanoethyl-N,N,N',N'-tertraisopropylphosphorodiamidite activated by the immobilized 4,5-DCI (C-1). 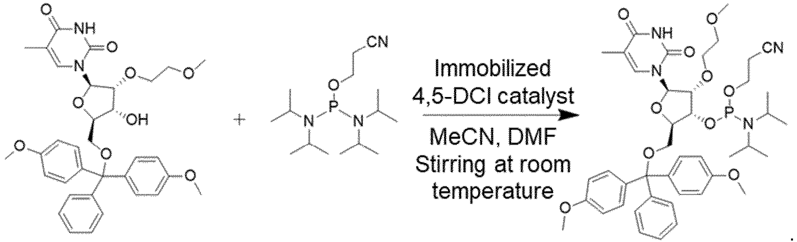 100 mg of immobilized 4,5-DCI resin was taken and added to 2 mL of acetonitrile, and 2-cyanoethyl-N,N,N',N'-tetraisopropylphosphorodiamidite (36 µL, 0.12 mmol) was added dropwise. 62 mg of the 5-methyluridine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methyluridine derivative was 91%.
3. A coupling reaction of an adenosine derivative and 2-cyanoethyl-N,N,N',N'-tertraisopropylphosphorodiamidite activated by the immobilized 4,5-DCI (C-1). 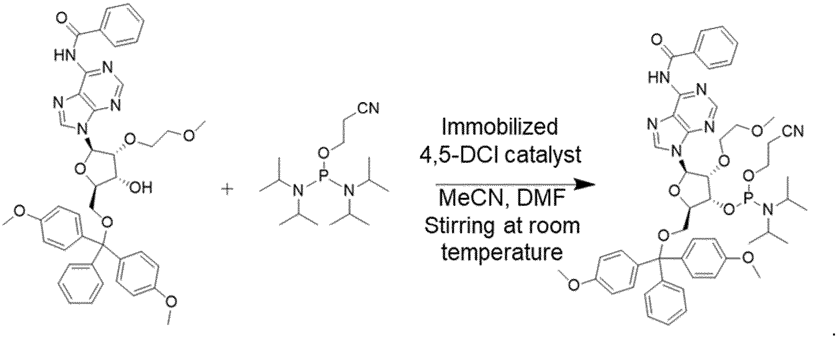 100 mg of immobilized 4,5-DCI resin was taken and added to 2 mL of acetonitrile, and 2-cyanoethyl-N,N,N',N'-tetraisopropylphosphorodiamidite (36 µL, 0.12 mmol) was added dropwise. 73 mg of the adenosine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the adenosine derivative was 90%.
4. A coupling reaction of a guanosine derivative and 2-cyanoethyl-N,N,N',N'-tertraisopropylphosphorodiamidite activated by the immobilized 4,5-DCI (C-1). 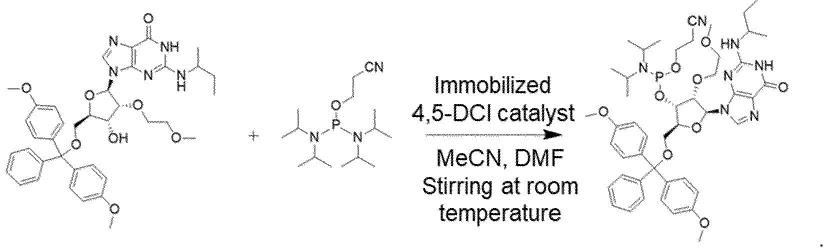 100 mg of immobilized 4,5-DCI resin was taken and added to 2 mL of acetonitrile, and 2-cyanoethyl-N,N,N',N'-tetraisopropylphosphorodiamidite (36 µL, 0.12 mmol) was added dropwise. 70 mg of the guanosine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the guanosine derivative was 93%.

### Embodiment 2

Under a nitrogen atmosphere, 0.4 g of divinylbenzene, 0.4 g of styrene, and 1.2 g of 2-ethenyl-4,5-DCI were put into a 25 mL two-necked flask, 10 mL of DMF was added as a solvent, and 25 mg of AIBN was added ultimately. A temperature was heated to 70 °C, and a system reaction was performed for 16 h. After overnight, heating was stopped. A product was filtered, washed with ethanol, and dried, so as to obtain 1.71 g of the product, and the obtained product was named as C-2.

A coupling reaction of a 5-methylcytidine derivative and 2-cyanoethyl-N,N,N',N'-tertraisopropylphosphorodiamidite activated by an immobilized 4,5-DCI (C-2):

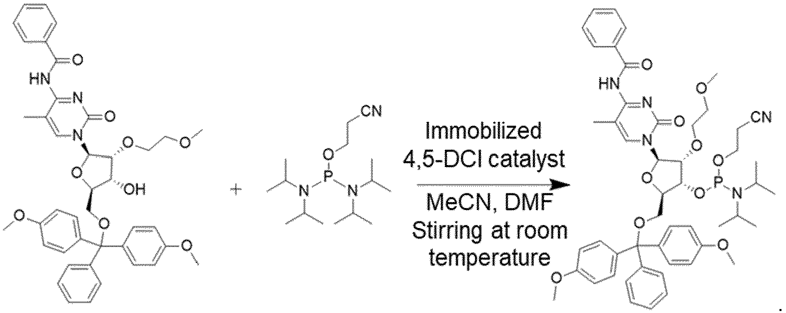

100 mg of the immobilized 4,5-DCI resin (C-2) was taken and added to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methylcytidine derivative was 86%.

### Embodiment 3

Under a nitrogen atmosphere, 0.6 g of divinylbenzene, 0.6 g of styrene, and 0.8 g of 2-ethenyl-4,5-DCI were put into a 25 mL two-necked flask, 10 mL of DMF was added as a solvent, and 25 mg of AIBN was added ultimately. A temperature was heated to 70 °C, and a system reaction was performed for 16 h. After overnight, heating was stopped. A product was filtered, washed with ethanol, and dried, so as to obtain 1.71 g of the product, and the obtained product was named as C-3.

A coupling reaction of a 5-methylcytidine derivative and 2-cyanoethyl-N,N,N',N'-tertraisopropylphosphorodiamidite activated by an immobilized 4,5-DCI (C-3):

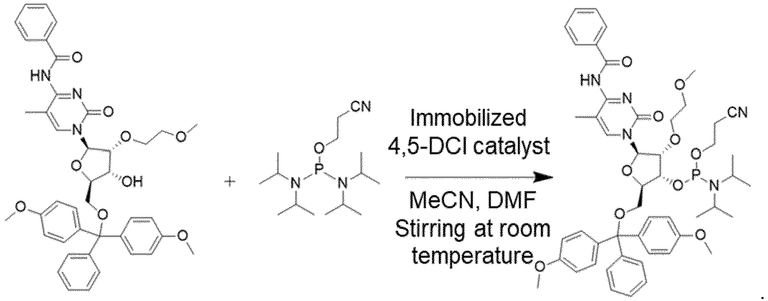

100 mg of the immobilized 4,5-DCI resin (C-3) was taken and added to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methylcytidine derivative was 85%.

### Embodiment 4

Under a nitrogen atmosphere, 1.0 g of divinylbenzene, 0.5 g of styrene, and 0.5 g of 2-ethenyl-4,5-DCI were put into a 25 mL two-necked flask, 10 mL of DMF was added as a solvent, and 25 mg of AIBN was added ultimately. A temperature was heated to 70 °C, and a system reaction was performed for 16 h. After overnight, heating was stopped. A product was filtered, washed with ethanol, and dried, so as to obtain 1.63 g of the product, and the obtained product was named as C-4.

A coupling reaction of a 5-methylcytidine derivative and 2-cyanoethyl-N,N,N',N'-tertraisopropylphosphorodiamidite activated by an immobilized 4,5-DCI (C-4):

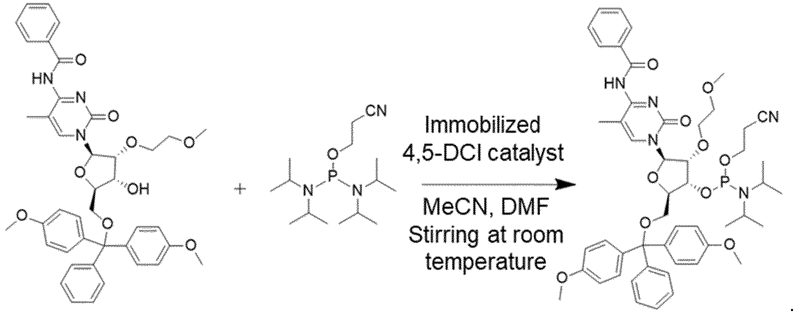

100 mg of the immobilized 4,5-DCI resin (C-4) was taken and added to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methylcytidine derivative was 42%.

### Embodiment 5

Under a nitrogen atmosphere, 0.5 g of divinylbenzene, 1.0 g of styrene, and 0.5 g of 2-ethenyl-4,5-DCI were put into a 25 mL two-necked flask, 10 mL of DMF was added as a solvent, and 25 mg of AIBN was added ultimately. A temperature was heated to 70 °C, and a system reaction was performed for 16 h. After overnight, heating was stopped. A product was filtered, washed with ethanol, and dried, so as to obtain 1.59 g of the product, and the obtained product was named as C-5.

A coupling reaction of a 5-methylcytidine derivative and 2-cyanoethyl-N,N,N',N'-tertraisopropylphosphorodiamidite activated by an immobilized 4,5-DCI (C-5).

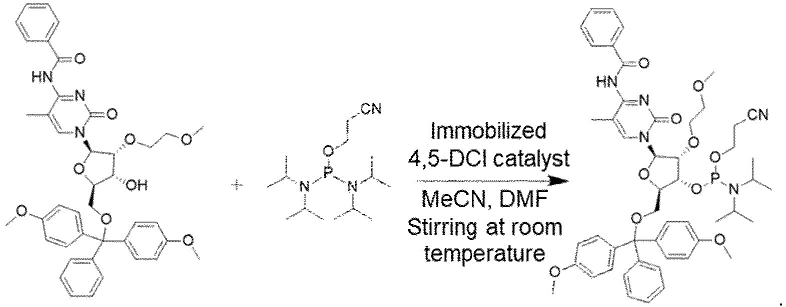

100 mg of the immobilized 4,5-DCI resin (C-5) was taken and added to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methylcytidine derivative was 35%.

### Embodiment 6

Under a nitrogen atmosphere, 0.75 g of divinylbenzene, 0.75 g of styrene, and 0.5 g of 2-ethenyl-4,5-DCI were put into a 25 mL two-necked flask, 10 mL of DMF was added as a solvent, and 25 mg of AIBN was added ultimately. A temperature was heated to 70 °C, and a system reaction was performed for 16 h. After overnight, heating was stopped. A product was filtered, washed with ethanol, and dried, so as to obtain 1.53 g of the product, and the obtained product was named as C-6.

A coupling reaction of a 5-methylcytidine derivative and 2-cyanoethyl-N,N,N',N'-tertraisopropylphosphorodiamidite activated by an immobilized 4,5-DCI (C-6).

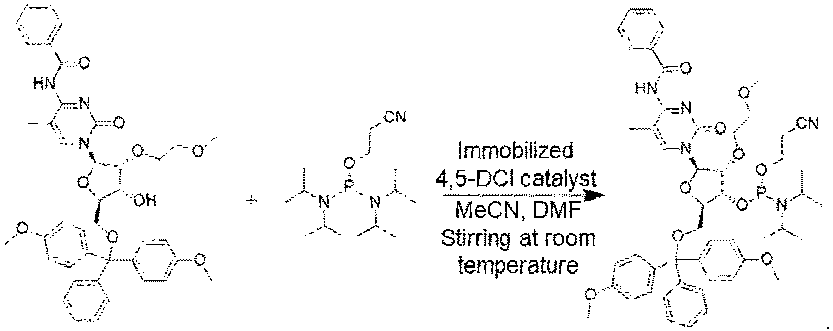

100 mg of the immobilized 4,5-DCI resin (C-6) was taken and added to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methylcytidine derivative was 38%.

### Comparative example 1

Under a nitrogen atmosphere, 1.0 g of divinylbenzene and 1.0 g of 2-ethenyl-4,5-DCI were put into a 25 mL two-necked flask, 10 mL of DMF was added as a solvent, and 25 mg of AIBN was added ultimately. A temperature was heated to 70 °C, and a system reaction was performed for 16 h. After overnight, heating was stopped. In this comparative example, since styrene was not added, a product obtained through polymerization was difficult to solidify.

### Embodiment 7

Under a nitrogen atmosphere, 0.5 g of divinylbenzene, 0.5 g of methyl methacrylate, and 1 g of 2-ethenyl-4,5-DCI were put into a 25 mL two-necked flask, 10 mL of DMF was added as a solvent, and 25 mg of AIBN was added ultimately. A temperature was heated to 70 °C, and a system reaction was performed for 16 h. After overnight, heating was stopped. A product was filtered, washed with ethanol, and dried, so as to obtain 1.32 g of the product, and the obtained product was named as C-7.

A coupling reaction of a 5-methylcytidine derivative and 2-cyanoethyl-N,N,N',N'-tertraisopropylphosphorodiamidite activated by an immobilized 4,5-DCI (C-7).

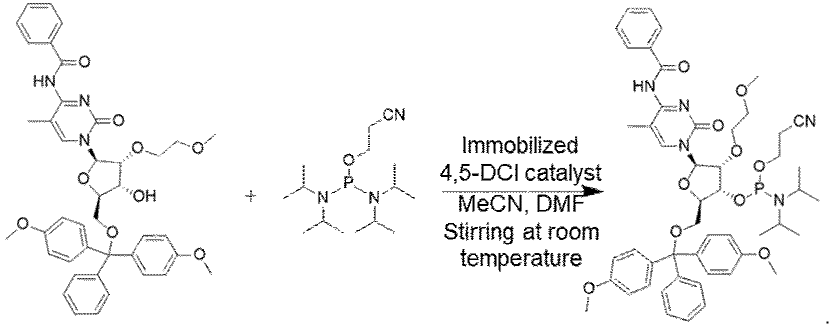

100 mg of the immobilized 4,5-DCI resin (C-7) was taken and added to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methylcytidine derivative was 76%.

### Embodiment 8

Under a nitrogen atmosphere, 0.5 g of ethylene glycol dimethacrylate, 0.5 g of styrene, and 1 g of 2-ethenyl-4,5-DCI were put into a 25 mL two-necked flask, 10 mL of N,N-dimethylformamide (DMF) was added as a solvent, and 25 mg of AIBN was added ultimately. A temperature was heated to 70 °C, and a system reaction was performed for 16 h. After overnight, heating was stopped. A product was filtered, washed with ethanol, and dried, so as to obtain 1.35 g of the product, and the obtained product was named as C-8.

A coupling reaction of a 5-methylcytidine derivative and 2-cyanoethyl-N,N,N',N'-tertraisopropylphosphorodiamidite activated by an immobilized 4,5-DCI (C-8).

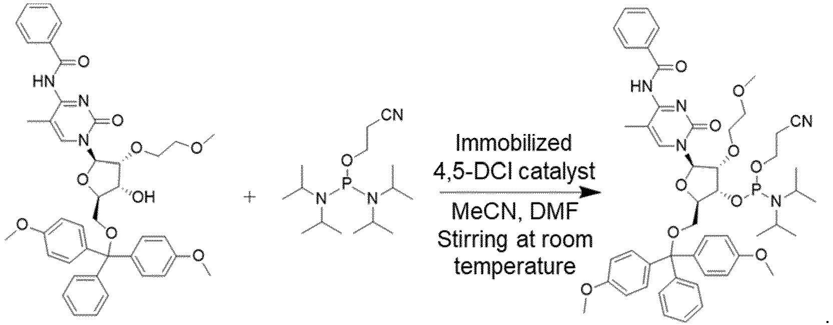

100 mg of the immobilized 4,5-DCI resin (C-8) was taken and added to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methylcytidine derivative was 81%.

### Embodiment 9

Under a nitrogen atmosphere, 0.5 g of ethylene glycol dimethacrylate, 0.5 g of methyl methacrylate, and 1 g of 2-ethenyl-4,5-DCI were put into a 25 mL two-necked flask, 10 mL of DMF was added as a solvent, and 25 mg of AIBN was added ultimately. A temperature was heated to 70 °C, and a system reaction was performed for 16 h. After overnight, heating was stopped. A product was filtered, washed with ethanol, and dried, so as to obtain 1.29 g of the product, and the obtained product was named as C-9.

A coupling reaction of a 5-methylcytidine derivative and 2-cyanoethyl-N,N,N',N'-tertraisopropylphosphorodiamidite activated by an immobilized 4,5-DCI (C-9).

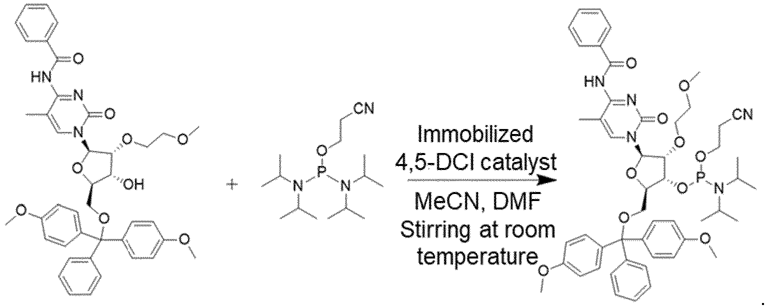

100 mg of the immobilized 4,5-DCI resin (C-9) was taken and added to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise and the reaction was overnight at room temperature. After the reaction was completed, analysis was performed through HPLC, and a phosphorylation product conversion rate of the 5-methylcytidine derivative was 75%.

### Comparative example 2: coupling reaction activated by free 4,5-DCI

1. A coupling reaction of a 5-methylcytidine derivative and 2-cyanoethyl-N,N,N',N'-tertraisopropylphosphorodiamidite activated by free 4,5-DCI. 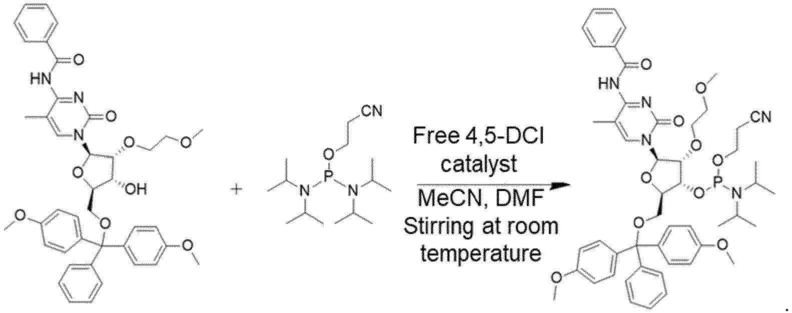 13 mg (0.11 mmol) of 4,5-DCI was taken and dissolved to 2 mL of acetonitrile, and 2-cyanoethyl-N,N,N',N'-tetraisopropylphosphorodiamidite (36 µL, 0.12 mmol) was added dropwise. 72 mg of the 5-methylcytidine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methylcytidine derivative was 97%.
2. A coupling reaction of a 5-methyluridine derivative and 2-cyanoethyl-N,N,N',N'-tertraisopropylphosphorodiamidite activated by free 4,5-DCI. 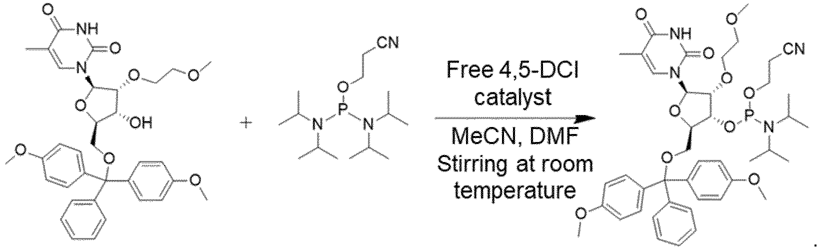 13 mg (0.11 mmol) of 4,5-DCI was taken and dissolved to 2 mL of acetonitrile, and 2-cyanoethyl-N,N,N',N'-tetraisopropylphosphorodiamidite (36 µL, 0.12 mmol) was added dropwise. 62 mg of the 5-methyluridine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the 5-methyluridine derivative was 95%.
3. A coupling reaction of an adenosine derivative and 2-cyanoethyl-N,N,N',N'-tertraisopropylphosphorodiamidite activated by free 4,5-DCI. 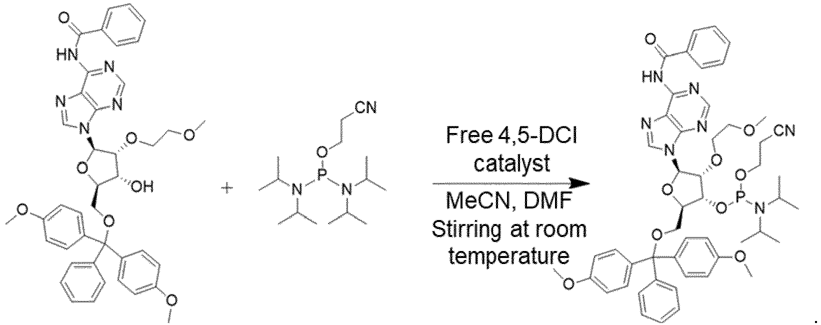 13 mg (0.11 mmol) of 4,5-DCI was taken and dissolved to 2 mL of acetonitrile, and 2-cyanoethyl-N,N,N',N'-tetraisopropylphosphorodiamidite (36 µL, 0.12 mmol) was added dropwise. 73 mg of the adenosine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the adenosine derivative was 95%.
4. A coupling reaction of a guanosine derivative and 2-cyanoethyl-N,N,N',N'-tertraisopropylphosphorodiamidite activated by free 4,5-DCI. 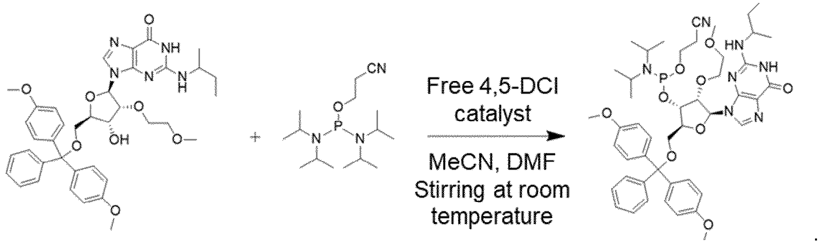 13 mg (0.11 mmol) of 4,5-DCI was taken and dissolved to 2 mL of acetonitrile, and 2-cyanoethyl-N,N,N',N'-tetraisopropylphosphorodiamidite (36 µL, 0.12 mmol) was added dropwise. 70 mg of the guanosine derivative (0.10 mmol) was taken and dissolved in 100 µL of the DMF, the above system was added dropwise, and the reaction was overnight at room temperature. After the reaction was completed, analysis was performed through HPLC, and a phosphorylation product conversion rate of the guanosine derivative was 96%.

Since the 4,5-DCI, substrate, phosphorus reagent, and the like were soluble in acetonitrile and DMF and insoluble in water, and were hardly removed by means of water washing and extraction after reaction, it was troublesome to use a nanofiltration membrane to remove impurities.

If the immobilized 4,5-DCI resin was used, a conversion rate of the substrate was basically equivalent to that of the small molecule 4,5-DCI, and the resin was separated only by means of centrifugation or filtration, thereby greatly simplifying impurity removing processes.

From the above descriptions, it might be seen that, the embodiments of the present disclosure achieved the following technical effects. The first comonomer and the second comonomer were used to perform the free radical copolymerization reaction on the 2-ethenyl-4,5-dicyanoimidazole, so as to prepare the immobilized 4,5-dicyanoimidazole. The immobilized 4,5-dicyanoimidazole could catalyze the phosphoramide and the hydroxyl to undergo the coupling reaction, and there was no significant decrease in the conversion rate of the substrate compared to activation using the 4,5-DCI.

## Claims

1. An immobilized 4,5-dicyanoimidazole, obtained through a free radical copolymerization reaction of a first comonomer, a second comonomer, and a third comonomer, wherein
the first comonomer is divinylbenzene or ethylene dimethacrylate; the second comonomer is styrene or methyl methacrylate; and the third comonomer is 2-ethenyl-4,5-dicyanoimidazole.

2. The immobilized 4,5-dicyanoimidazole according to claim 1, wherein in the immobilized 4,5-dicyanoimidazole, a mass ratio of the first comonomer, the second comonomer and the third comonomer is (10-50): (10-50): (10-80); a ratio of the total mass of the first comonomer and the second comonomer to the mass of the third comonomer is less than or equal to 9:1;
preferably, the mass ratio of the first comonomer, the second comonomer, and the third comonomer is (20-30): (20-30): (40-60); and
more preferably, the mass ratio of the first comonomer, the second comonomer and the third comonomer is 1:1:2.

3. The immobilized 4,5-dicyanoimidazole according to claim 1 or 2, wherein the solubility of the immobilized 4,5-dicyanoimidazole in all of N,N-dimethylformamide, ether, acetonitrile, alcohol, ester and chloralkane is less than 1 g/L;
preferably, an infrared spectrum of the immobilized 4,5-dicyanoimidazole has the following characteristic absorption: 3149-2488 cm⁻¹ of multiple peaks, 2242, 1911, 1728, 1644, 1574, 1510, and 1370 cm⁻¹; and a method for preparing an infrared test sample of the immobilized 4,5-dicyanoimidazole is a potassium bromide pellet method.

4. A method for preparing an immobilized 4,5-dicyanoimidazole, comprising:
applying a first comonomer and a second comonomer to undergo a free radical copolymerization reaction on 2-ethenyl-4,5-dicyanoimidazole, wherein
the first comonomer is divinylbenzene or ethylene dimethacrylate; and the second comonomer is styrene or methyl methacrylate.

5. The method according to claim 4, wherein in the free radical copolymerization reaction, a feeding mass of the first comonomer is 10-50 parts, a feeding mass of the second comonomer is 10-50 parts, and a sum of the feeding masses of the first comonomer and the second comonomer does not exceed 90 parts; a feeding mass of the 2-ethenyl-4,5-dicyanoimidazole is 10-80 parts;
preferably, the feeding mass of the first comonomer is 20-30 parts, the feeding mass of the second comonomer is 20-30 parts, and the feeding mass of the 2-ethenyl-4,5-dicyanoimidazole is 40-60 parts; and
more preferably, the feeding mass of the first comonomer is 25 parts, the feeding mass of the second comonomer is 25 parts, and the feeding mass of the 2-ethenyl-4,5-dicyanoimidazole is 50 parts.

6. The method according to claim 4, wherein the method comprises putting the first comonomer, the second comonomer and the 2-ethenyl-4,5-dicyanoimidazole in a reaction solvent to undergo the free radical copolymerization reaction, wherein the reaction solvent comprises one or more of tert-butyl methyl ether, acetonitrile or N,N-dimethylformamide; and
preferably, the free radical copolymerization reaction is undergone in an atmosphere of nitrogen or a rare gas.

7. The method according to claim 4, wherein a radical initiator of the free radical copolymerization reaction comprises one or more of 2,2'-azobis(2-methylpropionitrile), 2,2'-azobis(2,4-dimethyl)valeronitrile, or benzoyl peroxide;
preferably, a reaction temperature of the free radical copolymerization reaction ranges from 65 °C to 90 °C; and
preferably, a reaction time for the free radical copolymerization reaction ranges from 10 h to 20 h.

8. A coupling method, comprising: using the immobilized 4,5-dicyanoimidazole according to any of claims 1 to 3 or the immobilized 4,5-dicyanoimidazole prepared by the method for preparing immobilized 4,5-dicyanoimidazole according to any of claims 4 to 7 to activate phosphoramide and hydroxyl for coupling.

9. The coupling method according to claim 8, wherein a compound carrying the hydroxyl comprises a nucleoside protected by dimethoxytrityl or a deoxynucleoside protected by the dimethoxytrityl;
preferably, a compound carrying the phosphoramide comprises a phosphosamide compound; and
preferably, the phosphosamide compound comprises 2-cyanoethyl-N, N, N', N'-tertraisopropylphosphorodiamidite.

10. The coupling method according to claim 8 or 9, comprising: using the immobilized 4,5-dicyanoimidazole to undergo a continuous coupling reaction.

11. An application of the immobilized 4,5-dicyanoimidazole according to any one of claims 1 to 3, or method for preparing an immobilized 4,5-dicyanoimidazole according to any one of claims 4 to 7, or coupling method according to any of claims 8 to 10 in a coupling reaction of a phosphoramide and a hydroxyl and/or an oligonucleotide synthesis.
